# EUROPEAN PATENT SPECIFICATION

(11) **EP 2 981 319 B1**
(45) Date of publication and mention of the grant of the patent: **15.08.2018**
(21) Application number: 14712018.2
(22) Date of filing: 03.03.2014
(51) Int. Cl.: A61M 16/20

(54) **RESUSCITATOR ARRANGEMENTS AND FLOW MONITORING**
WIEDERBELEBUNGSANORDNUNGEN UND STRÖMUNGSÜBERWACHUNG
AGENCEMENTS DE RÉANIMATEUR ET SURVEILLANCE DE FLUX

(30) Priority: 04.04.2013 GB 201306067
(43) Date of publication of application: 10.02.2016
(73) Proprietor: Smiths Medical International Limited, Ashford, Kent TN25 4BF (GB)
(72) Inventor: BENNETT, Paul James Leslie, Marston Moretaine Bedfordshire MK43 0AQ (GB)
(74) Representative: Flint, Jonathan McNeill
(86) International application number: PCT/GB2014/000069
(87) International publication number: WO 2014/162107

(56) References cited:
- US-A1- 2003 230 307
- US-A1- 2010 024 824

## Description

This invention relates to resuscitator systems of the kind including a resuscitator, a gas flow conduit connected at one end to a gas outlet of the resuscitator, and a patient valve connected to the opposite end of the gas flow conduit and arranged to open to allow flow of gas from the gas flow conduit to a patient during an inspiratory phase and to close during an expiratory phase such that a major part of expired gas is prevented from flowing into the conduit and instead exits to atmosphere, the patient valve including a first valve element that is opened by expiratory gas pressure to allow gas to flow along an expiratory flow path.

Resuscitators are used to supply breathing gas to a patient who may not be breathing spontaneously. Portable resuscitators may take the form of a resilient bag that is squeezed manually to supply a volume of air to the patient, the bag refilling with air when it is released so that a new volume of air can be supplied. Alternatively, the resuscitator may be a mechanical device including a timing valve and various other controls and is connected to an oxygen cylinder, which both provides the breathing gas, or a part of this, and which may also provide the power to drive the components of the resuscitator. Examples of such resuscitators are described in US 2010/024824 A1, US 2003/230307 A1, GB 2174760, GB 2174609, EP 343818, EP 342883, EP 343824, GB 2282542, EP 691137, GB 2284159 and GB 2270629. These resuscitators are arranged to supply gas in a cyclical manner to the patient at a rate compatible with normal breathing. It is desirable to be able to provide the user of a resuscitator or ventilator with an indication of the expiratory tidal volume, that is, the volume of gas exhaled by the patient for each cycle. Conventionally flow is measured by means of a separate flow sensor connected in line between the patient valve and the patient. The flow sensor may have a flap element that moves between opposite sides of an aperture and is opened by flow in both directions but also provides an obstruction to both inspiratory and expiratory flow. Pressure lines opening on opposite sides of the flap element extend to respective pressure sensors so that an indication of the differential pressure across the flap element can be calculated and hence an indication can be derived of the flow rate. These devices have the disadvantage of obstructing both inspiratory and expiratory flow. The scope of the invention is as defined by the appended claims.

It is an object of the present invention to provide an alternative resuscitator or ventilator system.

According to the present invention there is provided a resuscitator system of the above-specified kind, characterised in that the patient valve arrangement includes a second valve element located in the expiratory flow path downstream of the first valve element, that the second valve element is opened by gas pressure passed by the first valve element during an expiratory phase, that the second valve element communicates with a chamber opening to atmosphere, that the patient valve arrangement includes two pressure monitoring channels extending from the patient valve along the gas flow conduit, the first channel opening to be exposed to gas pressure on an upstream side of the second valve element, the second channel opening to be exposed to gas pressure on a downstream side of the second valve element, and that the first and second channels extend to a differential pressure arrangement in the resuscitator that provides an output indication of expiratory gas flow from the differential pressure across the second valve element.

The differential pressure arrangement preferably includes processing for deriving an indication of expiratory tidal volume from the expiratory gas flow, such as by integrating the expiratory gas flow output over time. Preferably the gas flow conduit is provided by a length of flexible corrugated tubing. The second valve element preferably includes a flap valve. The resuscitator is preferably a gas-powered resuscitator.

A resuscitator system including a patient breathing circuit and its method of use, will now be described, by way of example, with reference to the accompanying drawings, in which:
- Figure 1: shows the system schematically and illustrates schematically the pressure monitoring sensors in the resuscitator;
- Figure 2A: is a cut-away perspective view of the patient valve assembly and the gas conduit showing gas flow during an expiratory phase;
- Figure 2B: is a cut-away perspective view of the patient valve assembly and conduit during an inspiratory phase;
- Figure 3: is a graph illustrating how differential pressure changes with inspiratory and expiratory flow;
- Figure 4: is a perspective view of the non-return flap valve in the outlet of the patient valve.

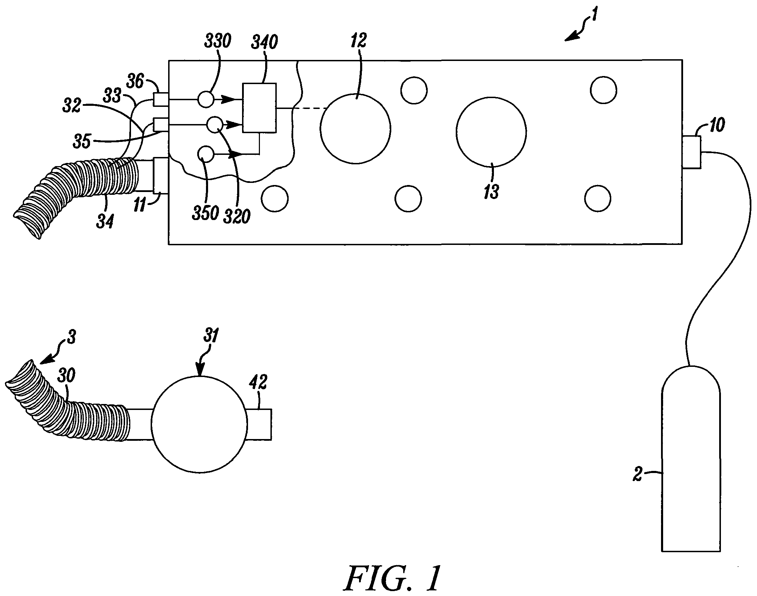

With reference first to Figure 1, the resuscitator system comprises a resuscitator unit 1, an oxygen cylinder 2 connected to a gas supply inlet 10 on the resuscitator, and a patient breathing circuit 3 connected to a ventilation gas outlet 11.

The resuscitator unit 1 is similar to that sold by Smiths Medical International Limited under the Parapac trade mark (Parapac is a Registered Trade Mark of Smiths Medical International Limited) with the addition of a function for calculating and indicating expiratory tidal volume on a display 12. The resuscitator unit 1 is of the mechanical, gas-powered kind where the gas from the oxygen cylinder 2 provides both the breathing gas, or a part of the breathing gas, and also provides the power to drive the components of the resuscitator such as the timing valve, air entrainment mixer and various other controls. The resuscitator 1 provides an adjustable, cyclical gas mixture during inspiratory breathing phases to the gas outlet 11, and from there to the breathing circuit 3.

The breathing circuit 3 includes two main components, namely a gas supply conduit 30 and a patient valve assembly 31. The gas supply conduit 30 takes the form of a flexible, corrugated tube 30' and also includes two small-bore pressure monitoring channels or lines 32 and 33 extending along the interior of the tube. At the machine end 34 of the breathing circuit 3 the pressure lines 32 and 33 connect with respective spigots 35 and 36, which make gas connection with respective pressure sensors 320 and 330 in the resuscitator 1 the function of which will be described later. The spigots 35 and 36 may be located within the gas outlet 11 or to one side of it.

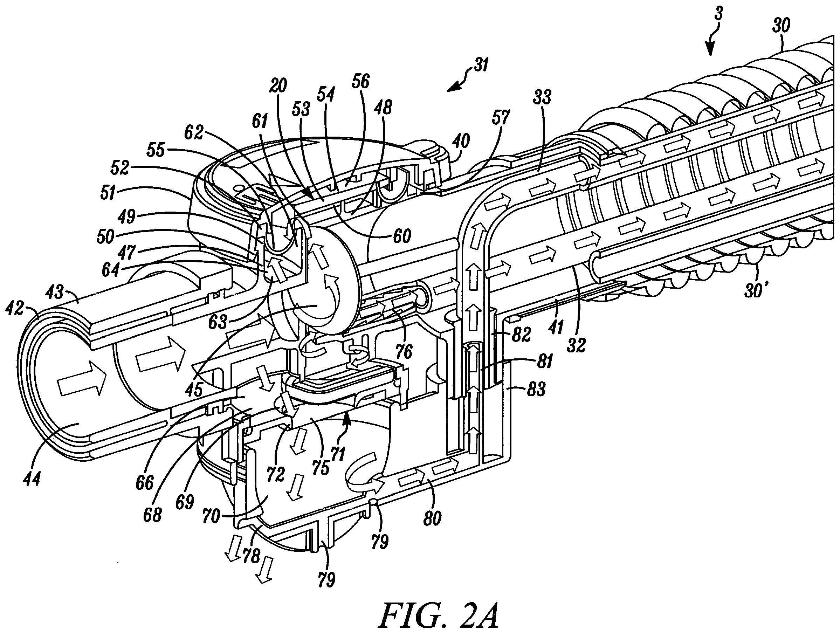

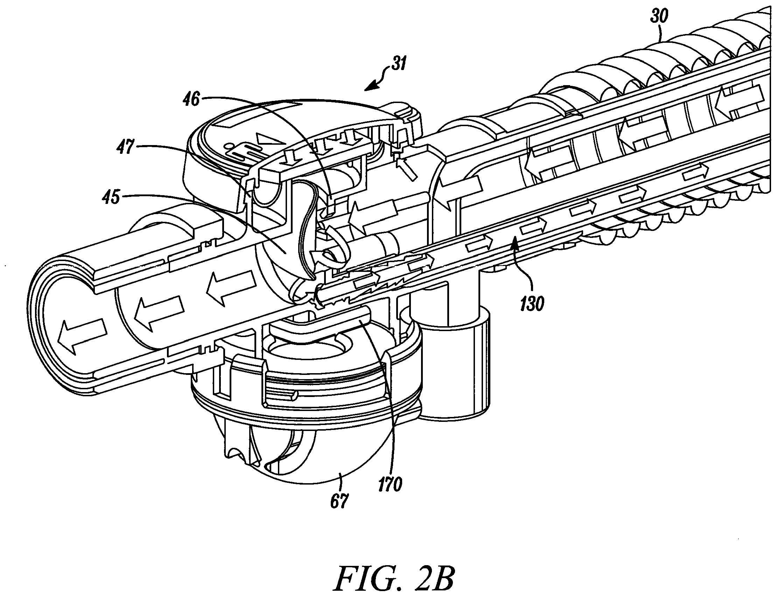

With reference to Figures 2A and 2B, the patient valve 31 includes an outer housing 40 of generally T shape in section having a tubular machine end extension 41 at one end onto which the patient end of the conduit 30 is fitted. At the opposite end of the housing 40 a patient end extension 42 extends forwardly in axial alignment with the machine end extension 41. The patient end extension 42 has a conventional luer tapered coupling surface 43. In use, the patient end extension 42 would be connected to a face mask, a tracheal tube, laryngeal mask or other airway by which gas can be supplied to and from the patient. Usually, an HME (heat and moisture exchange element) would be connected between the patient valve 31 and the face mask or the like. This helps conserve warmth and moisture within the patient's breathing passages by exchanging exhaled heat and moisture to inhaled gas. It also helps reduce the amount of moisture entering the patient valve 31 and so reduces the build up of condensation within it.

A through bore 44 extends along the housing 40 between the patient and machine end extensions 42 and 41. A flexible flap valve 45 of circular shape is mounted midway along the through bore 44 extending laterally of the bore and with its rear, machine side bearing on an annular valve seat 46 (shown more clearly in Figure 2B) formed around the inside of the housing 40. As shown in Figure 2B, the flap valve 45 flexes away from the valve seat 46 during the inspiratory phase, when gas pressure on the machine side of the valve exceeds that on the opposite side, to allow gas to flow along the bore through the patient valve 31 between opposite ends. Pressure on the patient side of the flap valve 45 is communicated to a pressure gauge 13 in the resuscitator unit 1 via a small-bore tube 130 extending along the conduit 30. This pressure is preferably also supplied to an alarm to provide a warning when pressure is outside predetermined limits. During the expiratory phase (Figure 2A) the gas pressure on the patient side of the flap valve 45 exceeds that on its opposite side so that it is forced against the valve seat 46, thereby preventing gas flowing past the flap valve.

An exhalation valve 20 is located in the valve housing 40 above the flap valve 45. This region the valve housing 40 is formed with an upwardly-projecting circular rim 47 around a base plate 48 extending in a plane parallel to the axis of the through bore 44. The rim 47 has a tapered lip 49 around its outside to which a corresponding lip 50 around the inside of a circular exhalation valve cap 51 is clipped to retain the cap in position on the housing 40. The cap 51 traps the outer edge 52 of an exhalation valve element 53 extending across the base plate 48. The exhalation valve element 53 is in the form of a circular resilient membrane having a flat central region 54 and a peripheral U-shape edge region 55 that is thinner and more flexible than the central region. The cap 51 defines a small volume upper chamber 56 above the valve element 53 that communicates with the bore 44 on the machine side of the flap valve 45 via a first, small opening 57 in the base plate 48 just outside the edge 52 of the valve element 53. A similar small volume lower chamber 60 is defined below the valve element 53 above the base plate 48. A second small opening 61 in the base plate 48 opens into the lower chamber 60 from the through bore 44 on the patient side of the flap valve 45.

The underside of the exhalation valve element 53 normally sits against a ledge 62 projecting from the base plate 48 at an outer edge of the lower chamber 60 to block escape of gas from the lower chamber. When the valve element 53 is lifted by a change in differential pressure across the element a gap is formed between the upper edge of the ledge 62 and the underside of the valve element opening into one end of an expiratory gas flow path 63. The gas flow path 63 is formed by a horizontal channel 64 extending laterally under the patient end side of the U-shape edge region 55 that opens into two vertical channels (not visible in the drawings) extending down opposite sides of the housing 40.

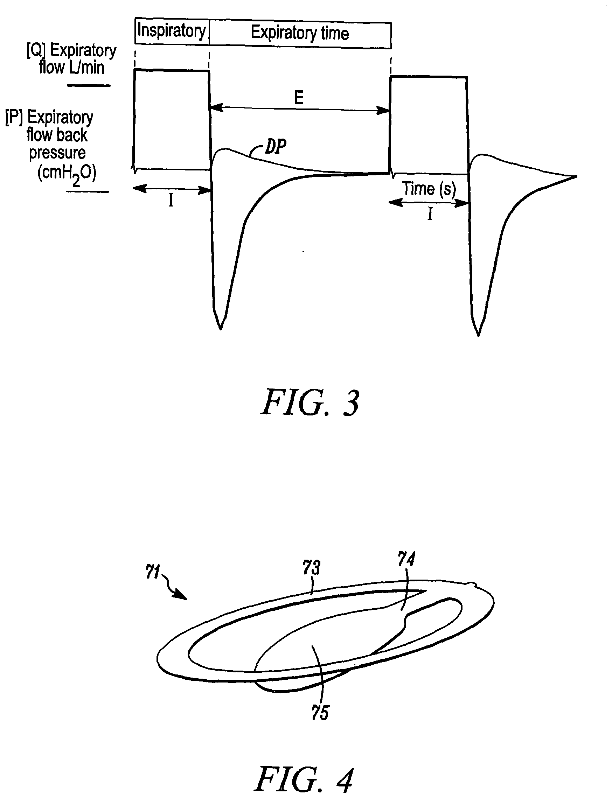

The vertical channels open into an upstream vent chamber 66 formed at the upper end of a domed outlet moulding 67 depending from the lower side of the patient valve assembly 31. The upstream chamber 66 has a floor 68 with a hole 69 through it opening into a lower, downstream vent chamber 70. The lower side of the hole 69 is covered by a non-return valve 71 that bears on a downwardly-projecting lip 72 around the hole forming a valve seat. The non-return valve 71 is, therefore, located downstream of the exhalation valve 20. The non-return valve 71 is shown most clearly in Fig 4 comprising an outer circular mounting ring 73 from which a short tongue 74 projects radially inwardly, supporting at its inner end a circular valve plate element 75 located concentrically within the ring 73. The non-return valve 71 is an integral, unitary, one-piece component stamped from a thin sheet of polyester material (such as Melinex 401CW). The valve 71 is attached to the under surface of the floor 68 by the mounting ring 73, with the plate element 75 positioned over the hcle 69. The construction and nature of the material of the non-return valve 71 is such that the plate element 75 only makes light contact with the valve seat 72 and readily opens when pressure in the upstream vent chamber 66 rises slightly. In its natural state, the plate element 75 may not, in fact, contact the valve seat 72 but, if a negative pressure were applied above the valve, such as caused by the patient inhaling, this would draw the plate element into sealing contact with the valve seat. One function of the non-return valve 71 is, therefore, to ensure that, if the patient inhales, gas is inhaled from the resuscitator and not via the expiratory gas flow path. The upstream vent chamber 66 also communicates with one of the pressure lines 32, which is fitted onto the outside of one end of a barbed spigot 76, the other end of which opens into the upstream vent chamber. In this way, pressure changes in the upstream vent chamber 66 are communicated to the pressure sensor 320 in the resuscitator 1. A partition plate 170 acts as a tray to collect condensation in the region of the open end of the pressure monitoring line 32 so that it can run away from the pressure line.

The downstream vent chamber 70 has an outlet aperture 78 opening to atmosphere and has two condensation drain holes 79 at its lower end to allow any condensation that might collect in the chamber to drain out. The chamber 70 also has a downstream pressure monitoring channel 80 communicating with a vertical tapered spigot 81. The patient end of the second pressure monitoring line 33 is fitted over the upper end of the spigot 81 and is held securely in place by a sleeve 82 pushed down around the line over the spigot and within a tubular side port 83 projecting from the patient end of the conduit 30.

The two pressure signals supplied along lines 32 and 33 are, therefore, derived from opposite sides of the non-return valve 71, which provides an obstruction or bluff body in the expiratory flow path 63 only.

In operation, during the inspiratory phase, the resuscitator unit 1 provides a positive gas pressure to its outlet 11 and hence to the machine end of the conduit 30. This gas pressure is communicated to the patient end of the conduit 30 and to the patient valve assembly 31. The positive pressure on the machine side of the flap valve 45 causes this to open so that gas can flow past the valve to the patient end 42 of the patient valve 31 and hence to the patient. Positive pressure on the machine end side of the flap valve 45 also causes gas to flow through the opening 57 to the upper chamber 56 above the exhalation valve element 53 ensuring that this is held down against the ledge 62 and closes the flow path via the other opening 61 to ensure that gas does not escape via the expiratory flow path 63. This phase is indicated by the letter "I" in the graph of Figure 3. Positive flow in this graph represents flow to the patient; negative flow represents flow from the patient. It can be seen that during the inspiratory phase "I" no gas is supplied to the upstream and downstream vent chambers 66 and 70 so the pressure on both sides of the non-return flap valve 71 is equal, giving a zero differential pressure output and hence a zero flow output.

During the expiratory phase "E", the resuscitator unit 1 stops supplying gas to the conduit 30 thereby allowing the patient to exhale and supply an expiratory gas stream to the patient end 42 of the patient valve assembly 31. This increases pressure on the patient side of the flap valve 45 forcing its opposite side into contact with the valve seat 46. Expiratory gas flows through the opening 61 into the lower chamber 60 beneath the expiratory valve element 53, thereby lifting the valve element and allowing gas to flow over the ledge 62 and into the expiratory gas flow path 63. Pressure, therefore, increases in the upstream vent chamber 66, opening the non-return valve 71 and allowing gas to flow into the downstream vent chamber 70 and to atmosphere via the outlet 78. This causes the pressure signals from opposite sides of the non-return valve 71 to be communicated along the pressure lines 32 and 33 to the sensors 320 and 330 in the resuscitator. The pressure in the upstream chamber 66 is higher than that in the downstream chamber 70 leading to a positive differential pressure as indicated by the line "DP" in Fig 3. This differential pressure is measured by a processor 340 in the resuscitator 1 and is converted to a flow rate measurement. The flow measurement is then converted to a total expiratory tidal volume measurement by integrating over the expiratory period and this is indicated on the display 12. The resuscitator 1 also includes a barometric pressure sensor 350 exposed to atmospheric pressure and connected with the processor 340 so that an appropriate correction can be made for changes in atmospheric pressure.

The small diameter of the pressure lines 32 and 33 makes them prone to blockage by particles, secretions or fluids (especially if these freeze during cold conditions). In order to prevent this, the resuscitator is arranged periodically automatically to supply a short positive pulse of gas to both lines such as once per inspiratory phase, or once every few inspiratory phases.

The present invention enables an indication of expiratory tidal volume to be provided without the need to obstruct the inspiratory gas flow path. Also, by incorporating the flow sensing within the patient valve itself it leads to a more compact configuration and avoids the need to connect separate components to the patient valve.

## Claims

1. A resuscitator system including a resuscitator (1), a gas flow conduit (30) connected at one end to a gas outlet (11) of the resuscitator, a patient valve (31) connected to the opposite end of the gas flow conduit and arranged to open to allow flow of gas from the gas flow conduit to a patient during an inspiratory phase and to close during an expiratory phase such that a major part of expired gas is prevented from flowing into the conduit and instead exits to atmosphere, the patient valve (31) including a first valve element (53) that is opened by expiratory gas pressure to allow gas to flow along an expiratory flow path (44), **characterised in that** the patient valve (31) includes a second valve element (71) located in the expiratory flow path downstream of the first valve element (53), that the second valve element (71) is opened by gas pressure passed by the first valve element (53) during an expiratory phase, that the second valve element (71) communicates with a chamber (70) opening to atmosphere, that the patient valve (31) includes two pressure monitoring channels (32 and 33) extending from the patient valve (31) along the gas flow conduit (30), the first channel (32) opening to be exposed to gas pressure on an upstream side of the second valve element (71), the second channel (33) opening to be exposed to gas pressure on a downstream side of the second valve element (71), and that the first and second channels (32 and 33) extend to a differential pressure arrangement (320, 330, 340) in the resuscitator (1) that provides an output indication of expiratory gas flow from the differential pressure across the second valve element (71).

2. A system according to Claim 1, **characterised in that** the differential pressure arrangement includes processing (340) for deriving an indication of expiratory tidal volume from the expiratory gas flow.

3. A system according to Claim 2, **characterised in that** the indication of expiratory tidal volume is derived by integrating the expiratory gas flow output over time.

4. A system according to any one of the preceding claims, **characterised in that** the gas flow conduit (30) is provided by a length of flexible corrugated tubing (30').

5. A system according to any one of the preceding claims, **characterised in that** the second valve element includes a flap valve (71).

6. A system according to any one of the preceding claims, **characterised in that** the resuscitator is a gas-powered resuscitator (1).

## Patentansprüche

1. Beatmungssystem mit einem Beatmungsgerät (1), einer Gasströmungsleitung (30), die an einem Ende mit einem Gasauslass (11) des Beatmungsgeräts verbunden ist, einem Patientenventil (31), das mit dem entgegengesetzten Ende der Gasströmungsleitung verbunden ist und angeordnet ist, um sich zu öffnen, um eine Strömung von Gas von der Gasströmungsleitung zu einem Patienten während einer Einatmungsphase zu ermöglichen, und sich während einer Ausatmungsphase zu schließen, so dass ein Hauptteil von ausgeatmetem Gas am Strömen in die Leitung gehindert wird und stattdessen in die Atmosphäre austritt, wobei das Patientenventil (31) ein erstes Ventilelement (53) umfasst, das durch den Ausatmungsgasdruck geöffnet wird, um zu ermöglichen, dass Gas entlang eines Ausatmungsströmungspfades (44) strömt, **dadurch gekennzeichnet, dass** das Patientenventil (31) ein zweites Ventilelement (71) umfasst, das im Ausatmungsströmungspfad stromabwärts des ersten Ventilelements (53) angeordnet ist, dass das zweite Ventilelement (71) durch den Gasdruck geöffnet wird, der durch das erste Ventilelement (53) während einer Ausatmungsphase geleitet wird, dass das zweite Ventilelement (71) mit einer Kammer (70) in Verbindung steht, die in die Atmosphäre mündet, dass das Patientenventil (31) zwei Drucküberwachungskanäle (32 und 33) umfasst, die sich vom Patientenventil (31) entlang der Gasströmungsleitung (30) erstrecken, wobei der erste Kanal (32) so mündet, dass er dem Gasdruck auf einer Stromaufwärtsseite des zweiten Ventilelements (71) ausgesetzt ist, der zweite Kanal (33) so mündet, dass er dem Gasdruck auf einer Stromabwärtsseite des zweiten Ventilelements (71) ausgesetzt ist, und dass der erste und der zweite Kanal (32 und 33) sich zu einer Druckdifferenzanordnung (320, 330, 340) im Beatmungsgerät (1) erstrecken, die eine Ausgangsangabe einer Ausatmungsgasströmung aus der Druckdifferenz über dem zweiten Ventilelement (71) bereitstellt.

2. System nach Anspruch 1, **dadurch gekennzeichnet, dass** die Druckdifferenzanordnung eine Verarbeitung (340) zum Ableiten einer Angabe des Ausatmungstidalvolumens aus der Ausatmungsgasströmung umfasst.

3. System nach Anspruch 2, **dadurch gekennzeichnet, dass** die Angabe des Ausatmungstidalvolumens durch Integrieren der Ausatmungsgasströmungsausgabe über die Zeit abgeleitet wird.

4. System nach einem der vorangehenden Ansprüche, **dadurch gekennzeichnet, dass** die Gasströmungsleitung (30) durch eine Länge eines flexiblen gerippten Schlauchs (30') vorgesehen ist.

5. System nach einem der vorangehenden Ansprüche, **dadurch gekennzeichnet, dass** das zweite Ventilelement ein Klappenventil (71) umfasst.

6. System nach einem der vorangehenden Ansprüche, **dadurch gekennzeichnet, dass** das Beatmungsgerät ein gasbetriebenes Beatmungsgerät (1) ist.

## Revendications

1. Système de réanimation comprenant un réanimateur (1), un conduit d'écoulement de gaz (30) connecté à une extrémité à une sortie de gaz (11) du réanimateur, une soupape patient (31) connectée à l'extrémité opposée du conduit d'écoulement de gaz et agencée pour s'ouvrir afin de permettre l'écoulement de gaz depuis le conduit d'écoulement de gaz à un patient pendant une phase inspiratoire et pour se fermer pendant une phase expiratoire de telle sorte qu'une majeure partie du gaz expiré est empêchée de s'écouler dans le conduit et sort plutôt dans l'atmosphère, la soupape patient (31) comporte un premier élément de soupape (53) qui est ouvert par pression gazeuse expiratoire pour permettre au gaz de s'écouler le long d'un chemin d'écoulement expiratoire (44), **caractérisé en ce que** la soupape patient (31) comporte un deuxième élément de soupape (71) situé dans le chemin d'écoulement expiratoire en aval du premier élément de soupape (53), que le second élément de soupape (71) est ouvert par pression gazeuse transmise par le premier élément de soupape (53) pendant une phase expiratoire, que le second élément de soupape (71) communique avec une chambre (70) s'ouvrant sur l'atmosphère, que la soupape patient (31) comprend deux canaux de surveillance de la pression (32 et 33) et s'étend à partir de la soupape patient (31) le long du conduit d'écoulement de gaz (30), la première ouverture du canal (32) devant être exposée à une pression gazeuse sur un côté amont du second élément de soupape (71), la seconde ouverture du canal (33) devant être exposée à une pression gazeuse sur un côté aval du second élément de soupape (71), et **en ce que** les premier et second canaux (32 et 33) s'étendent jusqu'à un agencement de pression différentielle (320, 330, 340) dans le réanimateur (1) qui fournit une indication de sortie du flux de gaz expiratoire à partir de la pression différentielle à travers le second élément de soupape. (71).

2. Système selon la revendication 1, **caractérisé en ce que** l'agencement de pression différentielle comporte un traitement (340) pour obtenir une indication du volume courant expiratoire à partir du flux de gaz expiratoire.

3. Système selon la revendication 2, **caractérisé en ce que** l'indication du volume courant expiratoire est obtenu en intégrant la sortie de flux de gaz expiratoire dans le temps.

4. Système selon l'une quelconque des revendications précédentes, **caractérisé en ce que** le conduit d'écoulement de gaz (30) est fourni par une longueur de tube flexible ondulé (30').

5. Système selon l'une quelconque des revendications précédentes, **caractérisé en ce que** le deuxième élément de soupape comporte une soupape à clapet (71).

6. Système selon l'une quelconque des revendications précédentes, **caractérisé en ce que** le réanimateur est un réanimateur à gaz (1).
